Europäisches Patentamt

European Patent Office

Office européen des brevets

(19)

(11) Publication number: **0 166 440**
A2

# (12) EUROPEAN PATENT APPLICATION

(21) Application number: **85107950.9**

(51) Int. Cl.⁴: **A 61 K 9/20**

(22) Date of filing: **27.06.85**

(30) Priority: **28.06.84 US 625438**

(71) Applicant: **G.D. Searle & Co., Box 1045, Skokie, Illinois 60076 (US)**

(72) Inventor: **Damani, Nalinkant Chunilal, 1004 Thurston Place, Arlington Heights, IL 60004 (US)**
Inventor: **Richards, Anthony Paul, 5714 West Ohio, Chicago, IL 60644 (US)**
Inventor: **Young, James George, 2729 Oak Avenue, Northbrook, IL 60062 (US)**

(43) Date of publication of application: **02.01.86**
**Bulletin 86/1**

(84) Designated Contracting States: **BE CH DE FR GB IT LI NL SE**

(74) Representative: **Beil, Hans Chr. et al, Beil, Wolff und Beil, Rechtsanwälte Adelonstrasse 58 Postfach 80 01 40, D-6230 Frankfurt am Main 80 (DE)**

(54) **Antacid composition.**

(57) The present invention relates to an antacid composition and to a process for preparing such compositons. In particular, the compositions comprise a unit dose amount of an antacid agent incorporated into a confectionery base.

EP 0 166 440 A2

Our No. 24 745

G.D. Searle & Co.
P.O.Box 1045
USA - Skokie, IL 60076

ANTACID COMPOSITION

## SUMMARY OF THE INVENTION

The present invention provides an antacid composition comprising from about 5 to 50% by weight of an active antacid agent and from about 50 to 95% by weight of a confectionery base comprising a confectionery sweetener and a plasticizer, wherein the composition has a porous microfine crystalline structure. In particular, the antacid compositions have a bulk density in a range of from about 0.8 to 1.4 g/cc.

In addition, the present invention provides a process for preparing such antacid compositions comprising:

a)    preparing an aqueous mixture of a confectionery sweetener and a plasticizer;

b)     heating the aqueous mixture until a liquidified solution forms and concentrating the liquidified solution at a temperature of from about 100-145°C until the moisture content of the liquidified solution is within a range of from about 2 to 10% by weight;

c)     cooling the liquidified solution until a flexible plastic-like confectionery base forms;

d)     incorporating an antacid agent into the flexible plastic-like confectionery base to form a product mass;

e)     aerating the product mass;

f)     dividing the aerated product mass into dosage units;

g)    curing the dosage units until a composition having a porous microfine crystalline structure is produced.

The present invention provides an antacid composition having a non-chewy, fast-dissolving texture thereby permitting the administration of an active antacid agent in a fine uniform particle size thus achieving the fast acting properties of a liquid.  The antacid compositions of the present invention may be considered to form a suspension in the mouth upon chewing.  In addition, the antacid compositions described herein offer the advantages of portability and exact dosage form associated with a tablet.


DETAILED DESCRIPTION OF THE INVENTION


The present invention relates to an antacid composition comprising an active antacid agent in a confectionery base.  As used herein, the term "confectionery base" refers to a composition comprising one or more confectionery sweeteners and plasticizers.  In accordance with the present invention, confectionery sweeteners include sugars effective in producing sweets and candies such as for example, sucrose, corn syrup, frutose and the like.  In addition, artificial sweeteners such as aspartame, cyclamates, saccharin, thalin, acesulfam K, sugar alcohols, and bulking agents such as, carbohydrates such as polydextrose, dextrin, starches, dextrose and the like, hydrogenated starch hydrolysates such as, lycasin and the like, or combinations thereof, may be used to replace all or a portion of the sugar. As used herein, the term "plasticizer" refers to polyhydric materials which thus allow the liquidified sugar to form a plastic or elastic flexible mass and retard crystallization until the curing process. Representative of plasticizers effective in the composition of the present invention include for example glycerine, propylene glycol, polyethylene glycol, sorbitol, hydrogenated starch hydrolysates and mixtures thereof, and the like. It is preferred to employ sorbitol or mixtures containing sorbitol as the plasticizer.

The antacid compositions of the present invention are prepared in accordance with the following procedure:

An aqueous solution comprising a confectionery sweetener and a plasticizer is heated until a liquidified solution forms. The liquidified solution is then "cooked" at a temperature within a range of form 120-145°C, more perferably from 129°C to 135°C, and if sorbitol is employed as the plasticizer, most preferably from 131-133°C, until the moisture content of the liquidified solution is within a range of from about 2-10% by weight.

Cooking the liquified solution at temperatures outside the range of 100°C-145°C would produce an unacceptable texture which would yield a soft, chewy, and uncured product at lower temperatures and a hard candy at higher temperatures. During the cooking step, nearly all water except for bound water is evaporated, resulting in a moisture content in the range of 2 to 10% and preferably 4 to 8% by weight. Upon obtaining the desired moisture content, cooking is discontinued and the liquidified confectionery base is allowed to cool until a flexible plastic film is formed. The cooling process may be accomplished using various conventional techniques. For example, the hot liquified solution may be poured onto a steel cooling table at ambient temperature resulting in the formation of a flexible plastic-like film. Upon cooling the film, preferably to a temperature within a range of 33-43°C at its approximate center, the liquified confectionery base has the consistency of a heavy gauge vinyl plastic film. Excessive disturbance of the confectionery base during this cooling operation is to be avoided as it may cause premature crystallization. Alternatively, the hot liquified confectionery base may be cooled using a continuous syrup cooker and either a cooling drum or a cooling steel conveyer belt. It should be noted that the temperature to which the confectionery base film is cooled does not appear to be critical. However, cooling of the film to a temperature only approximately 50°C or greater, may cause premature crystallization or affect the desired texture of the final product. The desired cooling temperature is readily ascertained by one of ordinary skill in the art and will depend in part upon the active antacid ingredient and the particular confectionery base used. In addition, it has

0166440

been found that the hot liquified confectionery base may be shocked cooled to a temperature within a range of from -10° to 25°C and then allowed to equilibrate to a temperature within a range of 33-43°C thereby resulting in the formation of a flexible plastic confectionery base. Following formation of the flexible plastic like mass, antacid agent is added to the mass. The antacid may be manually mixed or commercially available mechanical mixers may be utilized to incorporate the antacid agent. It should be noted that the mixing is preferably conducted at a temperature less than 60°C and more preferably within a range of 20-50°C in order to prevent premature crystallization or an unacceptable texture of a final product mix. When mechanical mixing devices are utilized, it is preferred to conduct the mixing within a temperature range of from 20 to 45°C. At all times during the process it is important to eliminate the presence of precrystallized material in the mixing chamber in view of the fact that this may induce crystallization of the final product. Following incorporation of the antacid agent into the flexible plastic-like confectionery base, the resulting product mix is aerated. As used herein, the term "aeration" refers to incorporating air into the product mass to provide a final porous structure upon curing. Aeration is conducted by simply manually mixing the antacid agent and plastic confectionery base or preferably using conventional techniques such as for example single blade mixers, confectioners taffy-pulling equipment, extruder mixers and the like. Aeration incorporates air into the product mix necessary to produce the required porous microfine structure, texture, mouth field, etc. In addition, it provides homogeneity with exact uniformity of the active antacid agent throughout the composition.

It should be noted that other additives, adjuvants, carriers and the like, such as glycerine, flavors, vitamins, coloring, mineral supplements, etc., may be added at any step of the process except if the material is heat sensitive, then the additive is preferably added during the aeration step.

The aerated product mass is then divided into dosage units. This may be accomplished using conventional techniques. It is preferable to form a rope and size the aerated product mass, dusting its exterior surface with a material to prevent sticking, such as corn starch, talc, magnesium stearate, powdered sugar and the like, and stretching and rolling the aerated product mass to a desired shape and size. On a mass production scale, a rope former and sizer generally rock the aerated product mass to form the desired shape and size. The thus-formed aerated product mass passes through sizing, rotating wheels to form a rope of uniform diameter. The rope mass is then cut into the desired uniform dose size tablets, cured and packaged. Curing techniques employed are readily ascertained by one of ordinary skill in the art.

The antacid compositions of the present invention may be characterized as having a microfine crystalline

0166440

structure and having a bulk density with a range of from 0.8 to 1.3 g/cc and preferably from 0.9 to 1.2 g/cc. The antacid compositions contain from about 5 to 50% by weight of an antacid agent; preferably from about 10 to 50% by weight, more preferably from about 15% to 40% by weight, and most preferably from about 20 to 30% by weight. The specific amount of antacid agent employed is readily ascertained by one of ordinary skill in the art and depends upon the particular active antacid agent employed, the desired dosage, the desired final tablet size, and the like. The confectionery base comprises from about 50-90% of a confectionery sweetener and from about 10-50% of a plasticizer. The specific amounts of confectionery sweetener and plasticizer employed depend upon the particular antacid agent, confectionery sweetener and plasticizer employed and are readily ascertained by one of ordinary skill in the art. It is preferred to employ a ratio of from 5:1 to 2:1 of confectionery sweetener: plasticizer.

As indicated by the above procedures, the process of the present invention involves the manufacture of a porous fast dissolving antacid composition as distinguished from compositions manufactured by compression or molding processes. In addition, the compositions of the present invention are considered non-chewy while having a porous microfine crystalline structure and are to be distinguished from chewy gum-type compositions. The antacid compositions may be in the form of tablets,

0166440

wherein the term "tablets" is used generically and is not intended to be limited to the form of the tablets nor does the term include tablets produced by compression or molding processes. The tablets produced in accordance with the present invention may be round, square, rectangular, oval, oblong, cylindrical, triangular, ring-shaped, etc.

Preferred antacid agents include, but are not limited to, active antacid agents such as calcium carbonate, aluminum hydroxide, magnesium oxide, magnesium carbonate, bismuth subsalicylate, aluminum oxide aluminum-magnesium hydroxide complexes such as megaldrate, and combinations thereof.

The particle size of the antacid agent is not critical and the desired particle size is readily ascertained by one of ordinary skill in the act. However, to improve taste, avoid chalkiness and to improve the speed of antacid activity, the finest possible size particles are employed. The preferred particle range is from sub-micron to about 5 micron size particles.

An advantage of the compositions and process of the present invention is that unlike in compression formed tablets, the majority of the particles of the antacid agent remain as separate particles within the confectionery base or become separate during consumption. The compositions of the present invention have significantly increased     acid neutralization activity. Most compression formed tablets comprise hard agglomerates of fine particles, thereby resulting in gritty, chalky taste and delay in the speed of acid neutralization activity.

The following examples describe in detail compounds illustrative of the present invention and methods which have been devised for their preparation.  It will be apparent to those skilled in the art that many modifications, both of materials and of methods, may be practiced without departing from the purpose and intent of this disclosure.

## Example 1

An antacid composition was prepared from the following ingredients in accordance with the process of the present invention:

| Ingredient | Parts by Weight |
|---|---|
| Calcium carbonate | 22.5 |
| Sucrose | 53.6 |
| Sorbitol Solution (70% Sorbitol by weight) | 13.4 |
| Glycerine | 0.4 |
| Spearmint Oil | 0.1 |
| Water | 10.0 |

The required amounts of sucrose, sorbitol solution and water were combined and heated to about 115°C with occasional stirring.  Upon formation of a liquidified confectionery base, the heating was continued without stirring until a temperature of approximately 132°C was obtained.  The liquidified confectionery base was maintained at 132°C for a period of time until the moisture content was reduced to less than 6% by weight.  During this cooking period, any crystals forming on the inner surface on the cooking vessel were redissolved into the liquidified solution. Upon reducing the moisture to an acceptable level, heating was discontinued and the hot liquidified confectionery base was poured onto a steel cooling table. The confectionery base was allowed to cool until the center temperature was about 38°C resulting in the formation of a flexible plastic-like confectionery base. The required amount of calcium carbonate was added in small increments to the plastic-like confectionery base with kneading. Following addition of the calcium carbonate, the resulting product mass was transferred onto conventional taffy pulling equipment and the product mass was pulled to mix and aerate the product mass. During aeration, the required amounts of glycerine and spearmint oil were added. The aeration process continued for approximately 3 minutes. The resulting aerated product mass was transferred to a suitable surface to form a rope. The exterior surface of the product mass was sprinkled with corn starch to prevent stickiness. The aerated product mass was stretched and rolled to form a rope having a diameter of approximately 3 inches. The rope was passed through a pinch roller to prepare the final size and shape of the antacid composition containing a unit dose of the calcium carbonate. The individual unit dose antacid compositions were dusted with corn starch and allowed to solidify. The final composition of the antacid compositions thus prepared was:

| Ingredient | Parts by Weight |
|---|---|
| Calcium carbonate | 25% |
| Sucrose | 59.8% |
| Sorbitol | 10.43% |
| Glycerine | 0.5% |
| Water | 4.17% |
| Spearmint Oil | 0.1% |

The density of the antacid compositions thus prepared was 1.11 g/cc.

As readily ascertained by one of ordinary skill in the art, the process of the present invention may be employed to prepare compositions containing other therapeutic agents, nutritional or dietary supplements in lieu of the active antacid agents. Illustrative of such active ingredients, but not limiting, include vitamins, mineral supplements, aspirin, aspirin substitutes, laxatives, anti-diarrheals and combinations thereof. Illustrative of the active ingredients include, for example, vitamins, calcium gluconate, calcium lactate, acetaminophen, ibuprofen, polycarbophil and salts thereof, glucomannon, dextromethorphan, chlorpheniramine, pseudoephedrin, and the like, as well as combinations and mixtures thereof.

Although this invention has been described with respect to specific modifications, the details thereof are not to be construed as limitations, for it will be apparent that various equivalents, changes and modification may be resorted to without departing from the spirit and scope thereof and it is understood that such equivalent embodiments are intended to be included therein.

What is claimed is:

1. An antacid composition comprising an antacid agent and a confectionery base comprising a confectionery sweetener and a plasticizer, wherein the composition has a porous microfine crystalline structure.

2. A composition according to Claim 1 comprising from about 5 to 50% by weight of an antacid agent.

3. A composition according to Claim 2 wherein the confectionery base comprises a confectionery sweetener and plasticizer in a ratio of from 5:1 to 2:1.

4. A composition according to Claim 3 having a bulk density in a range of from 0.8-1.3 g/cc.

5. A composition according to Claim 4 wherein composition contains from about 20 to 30% by weight of an antacid agent.

6. A composition according to Claim 5 wherein the plasticizer is sorbitol.

7. A composition according to Claim 6 wherein the active ingredient is an antacid agent selected from the class comprising calcium carbonate, aluminum hydroxide, bismuth subsalicylate, magnesium oxide, magnesium carbonate and aluminum- magnesium hydroxides.

-15-

8. A composition according to Claim 7 having a bulk density of from 1.0-1.2 g/cc.

9. A composition according to Claim 8 wherein the antacid agent is calcium carbonate.

10. A composition according to Claim 7 wherein the antacid agent is bismuth subsalicylate.

11. A composition according to Claim 9 wherein the confectionery sweetener is sucrose.

12. A process for preparing an antacid composition comprising from about 5 to 50% by weight of an antacid agent and from about 50 to 95% by weight of a confectionery base comprising a confectionery sweetener and a plasticizer, wherein the composition has a porous microfine crystalline structure, comprising:

0166440

a)    preparing an aqueous mixture of a confectionery
sweetener and a plasticizer;

b)    heating the aqueous mixture until a liquidified
solution forms and concentrating the liquidified
solution at a temperature of from about 100-145°C
until the moisture content of the liquidified solution
is within a range of from about 2-10% by weight;

c)    cooling the liquidified solution until a flexible
plastic-like confectionery base forms;

d)    incorporating an antacid agent into the flexible
plastic-like confectionery base to form a product mass;

e)    aerating the product mass;

f)    dividing the aerated product mass into dosage
units;

g)    curing the dosage units until a composition
having a porous microfine crystalline structure is
produced.

13. A process according to Claim 13 wherein the
composition comprises from about 20-30% by weight of
an antacid agent and a confectionery base comprising a
confectionary sweetener and a plasticizer wherein the
ration of confectionery sweetener: plasticizer is
within a range of from 5:1 to 2:1.

14. A process according to Claim 10 wherein the
plasticizer is sorbitol.

15. A process according to Claim 14 where in step (b) the liquidified confectionery base is maintained within a temperature range of from 129 -135  to reduce the moisture.

16. A process according to Claim 15 wherein the bulk density of the composition is in a range of from 0.8-1.3g/cc.

17. An antacid composition comprising from about 5 to 50% by weight of an antacid agent and from about 50 to 95% by weight of a confectionery base comprising from a confectionery sweetener and from a plastisizer, wherein the composition has a porous microfine crystalline structure, produced by a process comprising:

a)   preparing an aqueous mixture of a confectionery sweetener and a plasticizer;

b)   heating the aqueous mixture until a liquidified solution forms and concentrating the liquidified solution at a temperature of from about 100-145°C until the moisture content of the liquidified solution is within a range of from about 2-10% by weight;

c)    cooling the liquidified solution until a flexible plastic-like confectionery base forms;

d)    incorporating an antacid agent into the flexible plastic-like confectionery base to form a product mass;

e)    aerating the product mass;

f)    dividing the aerated product mass into dosage units;

g)    curing the dosage units until a composition having a porous microfine crystalline structure is produced.

18. A composition according to Claim 17 wherein the composition comprises from about 20-30% by weight of an antacid agent and a confectionery base comprising a confectionery sweetener and a plasticizer wherein the ratio of confectionery sweetener:  placticizer is within a range of from 5:1 to 2:1

19. A composition according to Claim 18 wherein the plasticizer is sorbitol.

20. A composition according to Claim 19 where the bulk density of the composition is in a range of from 0.8-1.3g/cc.